# FASCICULE DE BREVET EUROPEEN

(11) **EP 1 194 125 B1**
(45) Date de publication et mention de la délivrance du brevet: **25.04.2007**
(21) Numéro de dépôt: 00946045.2
(22) Date de dépôt: 30.06.2000
(51) Int. Cl.: A61K 9/16, A61K 9/50, A61K 9/20

(54) **PROCEDE DE FABRICATION DE GRANULES ENROBES A GOUT MASQUE ET LIBERATION IMMEDIATE DU PRINCIPE ACTIF**
VERFAHREN ZUR HERSTELLUNG EINES UMHÜLLTEN GESCHMACKMASKIERTEN GRANULATS MIT SOFORTIGER WIRKSTOFFFREISETZUNG
METHOD FOR MAKING GRANULES WITH MASKED TASTE AND INSTANT RELEASE OF THE ACTIVE AGENT

(30) Priorité: 08.07.1999 FR 9909047
(43) Date de publication de la demande: 10.04.2002
(73) Titulaire: ETHYPHARM, 78550 Houdan (FR)
(72) Inventeur: NOURI, Noureddine, F-06400 Cannes (FR); ZUCCARELLI, Jean-Marc, F-06600 Antibes (FR); CHAUVEAU, Charles, F-06560 Valbonne (FR); BRUNA, Etienne, F-28300 Jouy (FR)
(74) Mandataire: Vuillermoz, Bruno
(86) Numéro de dépôt international: PCT/FR2000/001855
(87) Numéro de publication internationale: WO 2001/003672

(56) Documents cités:
- EP-A- 0 525 389
- WO-A-99/09958
- US-A- 5 358 717

## Description

L'invention concerne un procédé de fabrication de granules enrobés à goût masqué et libération immédiate de principe actif. Elle se rapporte également aux granules enrobés de principe actif susceptibles d'être obtenus par ce procédé ainsi qu'à toute forme galénique incorporant lesdits granules enrobés.

Dans la suite de la description et dans les revendications, l'expression « *libération immédiate de principe actif »* signifie que la cinétique de libération de la molécule active n'est pas substantiellement modifiée par la formulation et/ou par les paramètres du procédé de fabrication (voir notamment document de l'agence européenne du médicament « note for guidance on modified release oral and transdermal dosage forms » du 22/04/1998). Dès lors, le profil de dissolution du principe actif dépend essentiellement de ses propriétés intrinsèques.

Pour obtenir une libération immédiate du principe actif, on a proposé dans le document EP-A-0 237 506 un procédé complexe de fabrication d'un granule à désintégration rapide selon lequel on prépare tout d'abord une solution d'un principe actif dans un mélange d'eau et d'alcanol, auquel on ajoute ensuite un agent émulsionnant, le mélange obtenu étant homogénéisé énergiquement. La composition alors obtenue est pulvérisée sur un lit de poudre comprenant un support inerte constitué d'une cellulose microcristalline et un agent désintégrant. L'agglomérat résultant est enfin séché puis présenté sous forme plus ou moins sphérique. Ce procédé n'a pas pour objet principal l'obtention de granules de principe actif à goût masqué.

En effet, il est bien connu qu'un certain nombre de principes actifs ont un goût désagréable de sorte qu'il est indispensable de masquer le goût de ces principes actifs au moins pendant leur séjour dans la cavité buccale, afin d'améliorer le confort et d'optimiser l'observance du traitement par le patient.

Une des solutions proposées consiste à enrober les particules de principe actif par un polymère cellulosique. Toutefois, même si le goût du principe actif présent dans les granules est masqué de façon satisfaisante, la faible perméabilité et la faible solubilité de tous les pH du polymère cellulosique conduit à une libération lente du principe actif, peu adapté à une cinétique de libération immédiate.

Pour résoudre ce problème, le Demandeur a proposé dans la demande de brevet français FR 2785539 publiée à la date 12.05.2000, d'enrober des particules d'ibuprofène par pulvérisation d'une solution à base d'éthylcellulose et d'hydroxypropylméthylcellulose, comprenant en outre un agent favorisant la solubilisation de l'ibuprofène.

Une autre solution consiste à enrober la particule de principe actif avec un polymère du type acrylique. Parmi ces polymères, on distingue les polymères pH dépendant, c'est à dire les polymères dont la solubilité dépend du pH, des polymères pH indépendant, c'est-à-dire les polymères dont la solubilité est indépendante du pH.

Les polymères pH dépendant en fonction de leur domaine de solubilité, peuvent entraîner une libération différée du principe actif jusque dans la partie distale de l'intestin. En d'autres termes, un tel enrobage est incompatible avec une libération immédiate du principe actif.

Les polymères acryliques pH indépendant sont, par définition, insolubles, de sorte que même s'ils donnent toute satisfaction en termes de masquage de goût, sont là encore peu adaptés, selon leur propriétés de perméabilité, à une libération immédiate du principe actif.

La mise en oeuvre de ce type de polymère est plus particulièrement décrite dans les documents US-A-4 726 966 et WO 98/47493.

Le document US-A-4 726 966 décrit un procédé de fabrication de microsphères d'ibuprofène par dissolution de particules d'ibuprofène dans un alcool aliphatique, puis recristallisation sous forme de microsphères à l'aide de différents solvants et résines acryliques. Ce procédé de fabrication, réalisé selon une technologie bien spécifique, permet d'obtenir un masquage du goût à priori satisfaisant.

De même le document WO 98/47493 décrit une composition pharmaceutique sous forme de granules enrobés d'un film polymérique du type acrylique, lequel, comme expressément indiqué, conduit à une libération retardée du principe actif.

De même, le document EP-A-0 255 725 décrit l'utilisation d'adjuvants de formulation (agents liants et désintégrants) dans la couche externe de granules à libération prolongée, présentés sous forme de comprimés. Dans ce cas, les adjuvants de formulation, dont la croscarmellose sodique réticulée et les dérivés de povidone, sont utilisés pour conférer une cohésion suffisante aux granules retards lors de la compression tout en assurant une désagrégation rapide du comprimé.

Le document EP-A-0 525 389 décrit un procédé d'obtention de comprimés multiparticulaires à désagrégation rapide, laquelle est conférée par l'utilisation de granules de principe actif enrobés avec notamment de la crospovidone. Ce composé est ici introduit dans le but de conférer une désagrégation rapide au comprimé tout en maintenant une cohésion suffisante. Des comprimés à désintégration rapide facilement compressibles sont connus des documents US 5358717 et EP 0525389.

Or, pour ces deux documents, la désagrégation rapide du comprimé ne signifie pas systématiquement libération immédiate de principe actif.

En d'autres termes, l'objectif de l'invention est de proposer un procédé de fabrication de granules enrobés, dont le goût du principe actif soit masqué, et dont la libération du principe actif soit immédiate, et ce, quelle que soit la nature du polymère d'enrobage.

Pour ce faire, l'invention propose un procédé de fabrication de granules enrobés à goût masqué, et libération immédiate du principe actif selon lequel :
- tout d'abord, on mélange à sec les constituants d'une poudre comprenant au moins le principe actif et un agent désintégrant granulaire ;
- on granule ensuite la poudre obtenue, en présence d'un mélange d'excipients comprenant au moins un agent liant apte à lier les particules entre elles pour obtenir des grains ;
- on enrobe alors les grains ainsi formés par pulvérisation d'une suspension comprenant au moins un agent d'enrobage et un agent désintégrant membranaire ;
- enfin, on sèche les granules enrobés obtenus.

Dans la suite de la description et dans les revendications, par l'expression « *agent désintégrant membranaire* », on désigne un excipient apte à augmenter la vitesse de désintégration de la couche d'enrobage des granules, obtenue à l'issue de l'étape d'enrobage.

De même, par l'expression *« agent désintégrant granulaire »*, on désigne un excipient apte à accélérer la vitesse de séparation des particules de principe actif entre elles après dissolution de la couche d'enrobage du granule.

En tant qu'agent désintégrant externe (AGM) et agent désintégrant interne (AGG), on utilise des "superdésintégrants" encore désignés désintégrants de haute efficacité. Les superdésintégrants sont largement connus de l'homme du métier, et plus particulièrement décrits dans la publication Journal of Pharmaceutical Sciences (Volume 85 n° 11 - Novembre 1996).

Dans le procédé de l'invention, les agents désintégrants granulaires et membranaires sont avantageusement choisis dans le groupe comprenant la carboxyméthylcellulose sodique, la crospovidone et le carboxyméthylamidon.

Le procédé de l'invention permet de résoudre de façon surprenante et inattendue les deux problèmes aux solutions antinomiques qui sont ceux d'obtenir le masquage du goût du principe actif par enrobage, tout en ne retardant pas pour autant la solubilisation du principe actif et ce, en intégrant à la fois au niveau granulaire et au niveau membranaire, outre la présence respectivement d'un agent liant et d'un agent d'enrobage, celle d'agents désintégrants (granulaire et membranaire).

De même, même si l'agent d'enrobage mis en oeuvre dans l'étape d'enrobage proprement dite, a pour fonction essentielle de parfaire l'enrobage définitif de chacun des grains, il peut cependant lier arbitrairement d'autres grains enrobés par mécanisme d'agglomération granulaire.

Dans une première forme de réalisation du procédé de l'invention, l'agent liant et l'agent d'enrobage sont choisis dans le groupe comprenant les polymères cellulosiques et les polymères acryliques.

Néanmoins, même si l'agent liant et l'agent d'enrobage sont choisis dans le même groupe de composés, ils diffèrent cependant l'un de l'autre de par leur fonction comme évoqué précédemment.

Parmi les polymères cellulosiques, on choisira avantageusement l'éthycellulose, l'hydroxypropylcellulose (HPC), la carboxyméthylcellulose (CMC) et l'hydroxypropylmethylcellulose (HPMC), seuls ou en mélange.

Parmi les polymères acryliques, on choisira avantageusement le copolymère amonio-méthacrylate (Eudragit^{®} RL ou RS), le polyacrylate (Eudragit^{®} NE), le copolymère acide méthacrylique (Eudragit^{®} L ou S), Eudragit^{®} étant une marque déposée par RÖHM.

Dans une forme avantageuse de réalisation, l'agent liant est de même nature que l'agent d'enrobage.

Pour accélérer d'avantage encore la libération du principe actif, la suspension d'enrobage comprend en outre un agent perméabilisant qui, de par ses propriétés de solubilité intrinsèques, provoque la perforation de l'enrobage membranaire, permettant ainsi de libérer le principe actif.

Parmi les agents perméabilisants utilisables, on distingue la povidone et ses dérivés, le polyéthylèneglycol, la silice, les polyols, et les polymères cellulosiques de faible viscosité.

En tant que polymère cellulosique de faible viscosité, on utilise par exemple les polymères du type hypromellose dont la viscosité est égale à 6 centipoises.

Pour permettre d'obtenir une action similaire au niveau granulaire, c'est à dire favoriser la libération des particules de principe actif liées au niveau des grains formés à l'issue de l'étape de granulation, le mélange d'excipient mis en oeuvre dans l'étape de granulation comprend en outre un agent perméabilisant du type de ceux décrits ci-avant.

Par ailleurs, pour optimiser le masquage du goût du principe actif, la suspension pulvérisée lors de l'étape d'enrobage comprend en outre un agent édulcorant.

De même, pour obtenir un masquage du goût du principe actif tout au long du processus de désintégration du granule enrobé, c'est à dire non seulement au niveau de la désagrégation progressive de la pellicule d'enrobage du granule, mais également au niveau de la séparation ultérieure des particules de principe actif, le mélange à sec de poudre initial peut comprendre également un agent édulcorant.

En tant qu'agent édulcorant, on peut utiliser l'aspartam, l'acésulfam de potassium, le saccharinate de sodium, la néohespéridine dihydrochalcone, le monoamonium glycyrrhizinate, les sucres et dérivés, ainsi que les polyols et dérivés, seuls ou en mélange.

De plus, pour conférer à la suspension d'enrobage et au mélange à sec de poudre initial des propriétés antistatiques, ceux-ci comprennent un agent antistatique choisi dans le groupe comprenant la silice précipitée ou colloïdale, et le talc.

Bien entendu, les étapes de granulation et d'enrobage peuvent être réalisées au sein d'appareils différents ou du même appareil et en présence, pour chaque étape, d'un mélange d'excipients de nature identique ou différente.

Dans une première forme de réalisation, le mélange à sec de poudre initial, les étapes de granulation, d'enrobage et de séchage sont effectuées en lit fluidisé.

Dans ce cas, le mélange de poudre initial est tout d'abord fluidisé, avant d'être granulé par pulvérisation sur ladite poudre du mélange d'excipient comprenant au moins l'agent liant, les grains obtenus étant ensuite enrobés par pulvérisation de la suspension d'enrobage, les granules enrobés formés étant enfin séchés dans le lit fluidisé.

Dans une forme de réalisation avantageuse, le mélange d'excipients mis en oeuvre lors de l'étape de granulation et la suspension d'enrobage mise en oeuvre lors de l'étape d'enrobage forment un seul mélange. Dans ce cas, l'étape de granulation se distinguera de l'étape de pulvérisation en faisant varier différents paramètres, tels que débit de pulvérisation du mélange et pression d'atomisation dudit mélange. Ainsi, une partie seulement du mélange d'excipients sera mise en oeuvre lors de l'étape de granulation tandis que l'autre partie sera mise en oeuvre lors de l'étape d'enrobage.

Ainsi, le débit de pulvérisation de la suspension d'enrobage est plus élevé lors de l'étape de granulation, que lors de l'étape d'enrobage, tandis que la pression d'atomisation de la suspension d'enrobage est moins élevée lors de l'étape de granulation que lors de l'étape d'enrobage.

En pratique, à l'échelle du laboratoire au sein d'un appareil à lit fluidisé par exemple du type GLATT GPCG1, lors de l'étape de granulation, le débit de pulvérisation de la suspension d'enrobage est compris entre 10 et 25 grammes/minute, et la pression d'atomisation est comprise entre 1 et 1,8 bars.

Lors de l'étape d'enrobage, le débit de pulvérisation de la suspension d'enrobage est compris entre 5 et 15 grammes/minute, tandis que la pression d'atomisation est comprise entre 1,5 et 2,5 bars.

Dans une forme de réalisation préférée, entre 10 et 20 % du mélange d'excipients est pulvérisé pendant l'étape de granulation, le complément à 100 % étant pulvérisé pendant l'étape d'enrobage.

En d'autres termes, et selon ce procédé avantageux, après avoir mélangé à sec le principe actif, l'agent désintégrant granulaire et avantageusement un agent édulcorant, on pulvérise sur le lit fluidisé une suspension d'excipients, comprenant l'agent désintégrant membranaire, l'agent d'enrobage, l'agent liant et l'agent perméabilisant en faisant varier le débit de pulvérisation et la pression d'atomisation de ladite suspension, de façon à obtenir tout d'abord une granulation puis ensuite un enrobage des grains formés.

Cependant dans une autre forme de réalisation, toujours au sein d'un même appareil, le premier mélange d'excipients est de nature différente du second, et ne contient notamment pas d'agent désintégrant membranaire.

Dans une autre forme de réalisation, l'étape de granulation et l'étape d'enrobage sont effectuées dans des appareils différents.

Ainsi, on pourra par exemple effectuer l'étape de granulation dans un granulateur à pales ou dans un granulateur à socs, tandis que l'étape d'enrobage pourra être effectuée en lit fluidisé. Bien entendu, de même que précédemment, le mélange d'excipients mis en oeuvre lors de l'étape de granulation et lors de l'étape d'enrobage pourra être identique ou différent.

L'invention se rapporte également aux granules enrobées susceptibles d'être obtenus par le procédé précédemment décrit.

Dans une forme de réalisation avantageuse, les granules enrobés de l'invention comprennent en poids du granule enrobé :
- de 5 à 70 % d'un polymère d'enrobage,
- de 0,5 à 15 % d'un agent désintégrant granulaire,
- de 1 à 20 % d'un agent désintégrant membranaire,
- de 1 à 20 % d'un agent perméabilisant.

Pour une concentration de polymère d'enrobage inférieure à 5 %, l'enrobage n'est pas suffisant pour permettre un bon masquage du goût. Pour une concentration supérieure à 70 %, la libération du principe actif est ralentie.

De même, pour une quantité d'agents désintégrants granulaire et membranaire inférieure à 1 %, la libération n'est pas immédiate. De même pour une concentration supérieure à 20 %, le masquage du goût n'est pas suffisant.

Parallèlement, pour une concentration en agent perméabilisant inférieure à 1 %, la libération est ralentie, tandis que pour une concentration supérieure à 20 %, le masquage du goût est insuffisant.

Pour permettre d'optimiser le masquage du goût, les granules comprennent en outre entre 1 et 20 % d'agent édulcorant.

Bien entendu, le polymère d'enrobage, agents désintégrants, agent perméabilisant et agent édulcorant sont choisis parmi les composés précédemment décrits.

Dans une forme avantageuse de réalisation, les granules enrobés de l'invention comprennent en poids du granule enrobé :
- de 10 à 40 % d'éthyl cellulose,
- de 3 à 10 % de crospovidone,
- de 2 à 10 % de polyéthylène glycol,
- de 2 à 10 % d'aspartam

Bien entendu, les granules enrobés fabriqués selon la procédé de l'invention peuvent être mis en oeuvre, en fonction du choix du polymère principal et des propriétés conférées à l'enrobage, au sein de toute formulation galénique adéquate.

Parmi ces formulations, on choisira avantageusement les comprimés du type comprimé multiparticulaire à délitement rapide tel que décrit par le demandeur dans le document FR-A-2 679 451, et obéissant à la monographie comprimé orodispersible de la Pharmacopée Européenne.

Cependant, les granules enrobés de l'invention peuvent être également mis en oeuvre au sein de comprimés dits "Fast dispersible", c'est-à-dire au sein de comprimés à hydrodispersibilité rapide susceptible de se déliter en un temps très court inférieur à 1 minute, de préférence inférieur à 15 secondes, dans un volume d'eau minimum, qui dépendra de la masse du comprimé.

Enfin, les granules enrobés peuvent être mis en oeuvre au sein de formulations galéniques classiques du type sachet, suspension, etc...

L'invention et les avantages qui en découlent ressortiront mieux des exemples de réalisation suivants à l'appui des figures annexées, dans lesquelles :
- la figure 1 est une représentation du profil de dissolution d'un comprimé multiparticulaire à délitement rapide d'életriptan fabriqué à partir de granules enrobés avec ou sans agent désintégrant granulaire et membranaire (AGG et AGM);
- la figure 2 est une représentation du profil de dissolution d'un comprimé multiparticulaire à délitement rapide d'ibuprofène fabriqué à partir de granules enrobés avec ou sans agent désintégrant (AGG et AGM) ;
- la figure 3 est une représentation du profil de dissolution d'un comprimé multiparticulaire à délitement rapide de prégabaline fabriqué à partir de granules enrobés avec ou sans agent désintégrant (AGG et AGM).

Les étapes de granulation et d'enrobage mises en oeuvre dans chacun des trois exemples suivants sont réalisées en lit fluidisé au sein d'un même appareil commercialisé par la société GLATT sous la dénomination GLATT GPCG1.

Par ailleurs, et pour chacun des exemples suivants, des tests de masquage de goût ont été réalisés auprès d'un échantillon d'individus. Les résultats sont indiqués en fonction de l'échelle suivante :
- goût principe actif non détecté
- goût principe actif détecté léger
- goût principe actif présent
- goût principe actif limite acceptable
- goût principe actif inacceptable

### EXEMPLE 1 : Granule enrobé à base d'életriptan intégré dans un comprimé du type multiparticulaire à délitement rapide

Comme déjà dit, les comprimés multiparticulaires à délitement rapide sont connus et plus particulièrement décrits dans le document du demandeur FR-A-2 679 451.

Pour la fabrication de ces comprimés, on procède tout d'abord à la préparation de granules enrobés de principe actif dont la composition est la suivante :

| **COMPOSITION GRANULE ENROBE** | | |
|---|---|---|
| principe actif | életriptan (sel) | 98,5 mg |
| | | équivalent à 80 g en principe actif de base) |
| AGG | croscarmellose sodique' | 4,90mg |
| agent d'enrobage | ethylcellulose | 20,40 mg |
| agent perméabilisant | polyoxyéthylène | |
| | glycol 6 000 | 4 mg |
| AGM | croscarmellose sodique | 3,70 mg |
| agent d'écoulement | | |
| /antistatique | silice précipitée | 1,40 mg |
| agent édulcorant | aspartam | 3,90 mg |

| | | |
|---|---|---|
| 1 : ACDISOL commercialisé par FMC. | | |

Les granulés sont fabriqués selon le procédé suivant. On prépare tout d'abord une solution de granulation en solubilisant 48 g d'éthylcellulose dans 273 g d'alcool éthylique.

Puis, on prépare ensuite une suspension d'enrobage en mélangeant dans 1 900 g d'alcool éthylique, 97 g d'éthylcellulose, 28,5 g de polyoléthylène glycol 6000, 26 g de croscarmellose sodique, 10 g de silice précipitée et 27,5 g d'aspartam, jusqu'à obtenir une suspension homogène.

On fluidise ensuite le mélange de poudre constituée de 700 grammes d'életriptan et 35 grammes d'ACDISOL.

On débute ensuite la granulation par pulvérisation de la solution de granulation pendant environ 15 à 20 mm à un débit de pulvérisation de 25 grammes/minute et une pression d'atomisation de la suspension de 0,8 bars.

On procède ensuite à l'enrobage proprement dit en pulvérisant la suspension d'enrobage pendant environ 1 h 30 à un débit de pulvérisation d'environ 15 à 20 grammes/minute, et une pression d'atomisation de la suspension de 1,5 bars.

Les granules enrobés ainsi obtenus sont ensuite formulés en comprimés multiparticulaires à délitement rapide dont la composition est la suivante :

| **COMPOSITION COMPRIME** | | |
|---|---|---|
| granulés enrobés | életriptan (sel) | 136,8 mg |
| | | équivalent de 80 g en principe actif de base) |
| agent de compression | mannitol | 575,20 mg |
| agent désintégrant | | |
| de comprimé | croscarmellose sodique | 24 mg |
| agent édulcorant | aspartam | 30 mg |
| aromatisant | arôme menthe réglisse | 10 mg |
| agent lubrifiant | stéarate de magnésium | 8 mg |

On fabrique les comprimés par tamisage de tous les excipients puis on homogénéise les granulés enrobés avec le mélange d'excipients dans un granulateur à socs. Les granules obtenus sont ensuite répartis et mis en forme sur une comprimeuse rotative. La dureté des comprimés obtenus est de 30 N environ.

### Résultat :

### • masquage de goût

Les tests gustatifs réalisés sur les comprimés sont satisfaisants : goût principe actif non détecté.

### • profil de libération de l'életriptan

On réalise une cinétique de dissolution des comprimés d'életriptan fabriqués dans un appareil du type I, selon la Pharmacopée Européenne 3^{ème} édition, en milieu acide HCL 0,1 N, avec un volume de dissolution de 900 ml. Le nombre de tours de pales par minute est égal à 100.

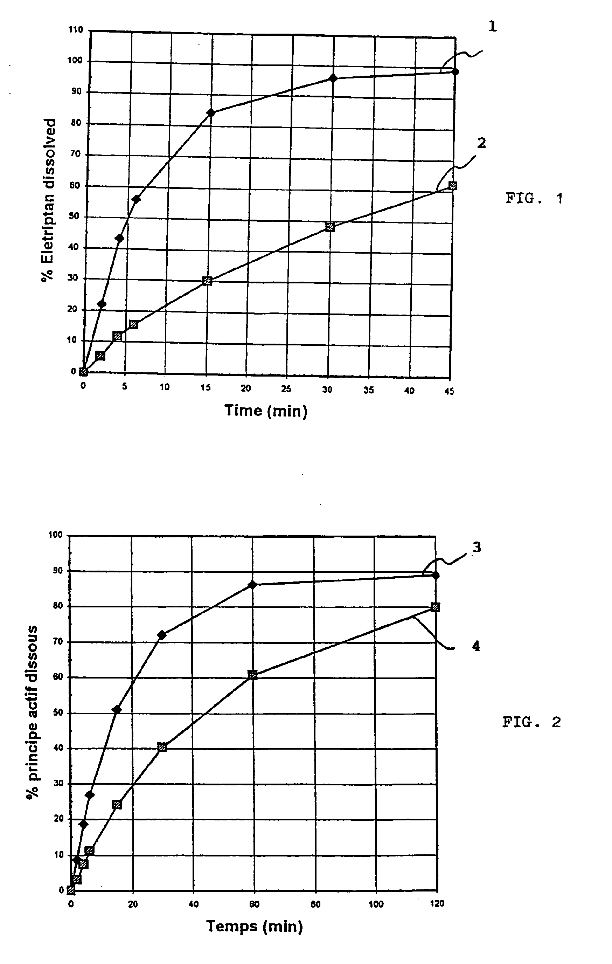

La figure 1 représente le profil de dissolution de comprimés d'életriptan avec agents désintégrants (AGG et AGM) (courbe 1) et sans agent désintégrant (courbe 2). Comme le montre cette figure, la présence d'agents désintégrants introduits non seulement au niveau de la granulation (AGG), mais également au niveau de l'étape d'enrobage des granules (AGM) permet d'obtenir une libération immédiate du principe actif.

### EXEMPLE 2 : Granule enrobé à base d'ibuprofène intégré dans un comprimé

### du type multiparticulaire à délitement rapide

On prépare des granules d'ibuprofène, dont la composition est la suivante :

| **COMPOSITION GRANULES** | | |
|---|---|---|
| principe actif | ibuprofène | 200 mg |
| agent désintégrant granulaire | | |
| (AGG) | croscarmellose sodique | 16 mg |
| agent édulcorant | aspartame | 27,5 mg |
| agent d'écoulement | | |
| /antistatique | silice précipitée | 12,20 mg |
| agent d'enrobage | éthylcellulose | 35 mg |
| agent perméabilisant | hypromellose¹ | 8 mg |
| agent désintégrant | | |
| membranaire (AGM) | croscarmellose sodique | 1,33 mg |

| | | |
|---|---|---|
| 1 : pharmacoat 606 commercialisé par Shinetsu | | |

Dans cet exemple, le mélange d'excipient mis en oeuvre lors de l'étape de granulation et la suspension d'enrobage mise en oeuvre lors de l'étape d'enrobage forment un seul mélange.

Ledit mélange est une suspension obtenue en mélangeant dans l'alcool éthylique, l'éthylcellulose, l'agent désintégrant membranaire, 80 % de la silice précipitée, 30 % de l'aspartame, jusqu'à obtenir une suspension homogène.

On fluidise ensuite le mélange de poudre constitué de l'ibuprofène, de l'agent désintégrant granulaire, de 70 % de l'aspartame et de 20 % de la silice précipitée.

On débute ensuite la granulation par pulvérisation du mélange pendant environ 15 à 20 minutes à une pulvérisation de 25 grammes par minute et une pression d'atomisation de la suspension de 0,8 bars.

On procède ensuite à l'enrobage proprement dit en pulvérisant le reste du mélange pendant environ 1 h 30 à un débit de pulvérisation de 15 à 20 grammes par minute et une pression d'atomisation de la suspension de 1,5 bars.

15 % du mélange est pulvérisé pendant l'étape de granulation, le complément à 100 % étant pulvérisé pendant l'étape d'enrobage.

Les granules obtenus sont ensuite formulés en comprimés multiparticulaires à délitement rapide, dont la composition est la suivante :

| **COMPOSITION COMPRIMES** | | |
|---|---|---|
| granules enrobés | | 300 mg |
| agent diluant | mannitol | 344 mg |
| agent désintégrant de | | |
| comprimés | croscarmellose sodique | 21 mg |
| agent d'écoulement | silice précipitée | 7 mg |
| agent édulcorant | aspartam | 20 mg |
| aromatisant | arôme menthe | 4 mg |
| agent lubrifiant | stéarate de magnésium | 4 mg |

### Résultats :

### - masquage du goût

Le masquage du goût est satisfaisant : goût principe actif non détecté.

### - Profil de libération

La cinétique de dissolution est réalisée dans un appareil de type II selon la Pharmacopée Européenne 3^{ème} édition.

On a représenté sur la figure 2 le profil de dissolution de comprimés d'ibuprofene avec ou sans agent de désintégration (respectivement courbes 3 et 4).

### EXEMPLE 3 : Granule enrobé à base de prégabaline intégré dans un comprimé du type multiparticulaire à délitement rapide

On prépare des granules de pregabaline, dont la composition est la suivante :

| **COMPOSITIONS GRANULES** | | |
|---|---|---|
| principe actif | pregabaline | 150 mg |
| AGG | crospovidone¹ | 6,43 mg |
| agent édulcorant | acesulfame de potassium² | 7,5 mg |
| agent d'écoulement | | |
| /antistatique | silice précipitée | 4,28 mg |
| agent d'enrobage | éthylcellulose | 39,64 mg |
| AGM | crospovidone | 6,43 mg |

| | | |
|---|---|---|
| 1 : kollidon CL commercialisé par BASF 2 : Sunett commercialisé par Nutrinova | | |

Le procédé de fabrication des granules enrobés est similaire à celui de l'exemple 2 à la différence près qu'on mélange à sec le PA, l'AGG, la moitié de la masse d'agent édulcorant et la moitié de la masse d'agent antistatique.

Les granulés obtenus sont ensuite formulés en comprimés multiparticulaires à délitement rapide dont la composition est la suivante :

| **COMPOSITION COMPRIME** | | |
|---|---|---|
| granulés enrobés | | 150 mg |
| agent de compression | mannitol | 474 mg |
| agent désintégrant | crospovidone' | 80 mg |
| agent édulcorant | aspartam | 14 mg |
| agent aromatisant | arôme | 8 mg |
| agent lubrifiant | stéarate de magnésium | 8 mg |

| | | |
|---|---|---|
| 1 : Kollidon CL commercialisé par BASF | | |

Les comprimés multiparticulaires sont fabriqués selon un procédé identique à l'exemple 1.

### Résultats :

### • masquage de goût

Les tests gustatifs réalisés sur les comprimés sont satisfaisants.

### • profil de libération de la prégabaline.

La cinétique de dissolution est réalisée dans un appareil de type II selon la Pharmacopée Européenne 3^{ème} édition.

On réalise sur les comprimés obtenus une cinétique de dissolution dans un appareil du type II en milieu HCL 0,06 N, avec un volume de dissolution de 900 ml, avec une vitesse des pales de 50 tours par minute.

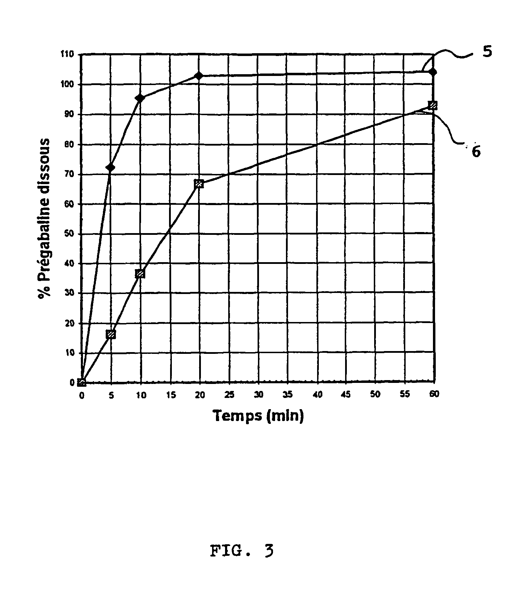

On a représenté sur la figure 3 le profil de dissolution de comprimés de prégabaline comprenant un agent désintégrant (courbe 5) et celui de comprimé de prégabaline sans agent désintégrant (courbe 6).

La courbe 5 montre que la prégabaline est libérée immédiatement.

Les avantages de l'invention ressortent bien de la description.

On notera en particulier la possibilité d'obtenir une formulation dont le goût du principe actif soit masqué, et ce sans pour autant retarder la libération du principe actif.

Par ailleurs, l'étape de granulation et d'enrobage caractéristique du procédé de l'invention peut être mise en oeuvre au sein d'appareillages différents ou au sein d'un même appareillage et ce, avec au choix des mélanges d'excipients identiques ou différents.

En outre, les granules enrobés obtenus peuvent être incorporés dans toute forme galénique adéquate du type gélule, comprimé multiparticulaire, comprimé, sachet, etc...

## Revendications

1. Procédé de fabrication de granules enrobés à goût masqué, et libération immédiate du principe actif selon lequel :
• tout d'abord, on mélange à sec les constituants d'une poudre comprenant au moins le principe actif et un agent désintégrant granulaire ;
• on granule ensuite la poudre obtenue, en présence d'un mélange d'excipients comprenant au moins un agent liant apte à lier les particules entre elles pour obtenir des grains et ne contenant pas d'agent désintégrant membranaire ;
• on enrobe alors les grains formés par pulvérisation d'une suspension comprenant au moins un agent d'enrobage et un agent désintégrant membranaire ;
• enfin, on sèche les granules enrobés obtenus.

2. Procédé de fabrication de granules enrobés à goût masqué, et libération immédiate du principe actif selon lequel :
• tout d'abord, on mélange à sec les constituants d'une poudre comprenant au moins le principe actif et un agent désintégrant granulaire ;
• on granule ensuite la poudre obtenue puis on enrobe les grains formés, en présence d'un même mélange d'excipients comprenant au moins un agent liant apte à lier les particules entre elles pour obtenir des grains ; au moins un agent d'enrobage et un agent désintégrant membranaire ; le débit de pulvérisation du mélange d'excipients étant plus élevé lors de l'étape de granulation, que lors de l'étape d'enrobage, et la pression d'atomisation du mélange d'excipients étant moins élevée lors de l'étape de granulation que lors de l'étape d'enrobage.
• enfin, on sèche les granules enrobés obtenus.

3. Procédé de fabrication selon la revendication 2, **caractérisé en ce qu'**entre 10 et 20 % du mélange d'excipients est pulvérisé pendant l'étape de granulation, le complément étant pulvérisé pendant l'étape d'enrobage.

4. Procédé de fabrication de granules enrobés selon l'une des revendications 1 à 3, **caractérisé en ce que** les agents désintégrants granulaire et membranaire sont des désintégrants de haute efficacité choisis dans le groupe comprenant la carboxyméthylcellulose sodique, la crospovidone et le carboxyméthylamidon.

5. Procédé de fabrication de granules enrobés selon l'une des revendications 1 à 3, **caractérisé en ce que** l'agent liant et l'agent d'enrobage sont choisis dans le groupe comprenant les polymères cellulosiques et les polymères acryliques.

6. Procédé de fabrication de granules enrobés selon la revendication 5, **caractérisé en ce que** le polymère cellulosique est choisi dans le groupe comprenant l'éthycellulose, l'hydroxypropylcellulose (HPC), la carboxyméthylcellulose (CMC) et l'hydroxypropylmethylcellulose (HPMC), seuls ou en mélange.

7. Procédé de fabrication de granules enrobés selon la revendication 5, **caractérisé en ce que** le polymère acrylique est choisi dans le groupe comprenant les polymères acryliques et les polymères méthacryliques, le copolymère amonio-méthacrylate, le polyacrylate, le copolymère acide méthacrylique.

8. Procédé de fabrication de granules enrobés selon la revendication 1, **caractérisé en ce que** la suspension comprend en outre un agent perméabilisant.

9. Procédé de fabrication de granules enrobés selon la revendication 1, **caractérisé en ce que** le mélange d'excipients mis en oeuvre dans l'étape de granulation comprend en outre un agent perméabilisant.

10. Procédé de fabrication selon l'une des revendications 2 ou 3, **caractérisée en ce que** le mélange d'excipients comprend en outre un agent perméabilisant.

11. Procédé de fabrication de granules enrobés selon l'une des revendications 8 à 10, **caractérisé en ce que** l'agent perméabilisant est choisi dans le groupe comprenant la povidone et ses dérivés, le polyéthylèneglycol, la silice, les polyols, et les polymères cellulosiques de faible viscosité.

12. Procédé de fabrication de granules enrobés selon la revendication 1, **caractérisé en ce que** la suspension pulvérisée lors de l'étape d'enrobage comprend en outre un agent édulcorant.

13. Procédé de fabrication de granules enrobés selon l'une des revendications 1 à 3, **caractérisé en ce que** le mélange à sec de poudre initial comprend en outre un agent édulcorant.

14. Procédé de fabrication selon l'une des revendications 2 ou 3, **caractérisé en ce que** le mélange d'excipients comprend en outre un agent édulcorant.

15. Procédé de fabrication de granules enrobés selon l'une des revendications 12 à 14, **caractérisé en ce que** l'agent édulcorant est choisi dans le groupe comprenant l'aspartam, l'acésulfam de potassium, le saccharinate de sodium, la néohespéridine dihydrochalcone, le monoamonium glycyrrhizinate, les sucres et dérivés, ainsi que les polyols et dérivés, seuls ou en mélange.

16. Procédé de fabrication de granules enrobés selon l'une des revendications 1 à 3, **caractérisé en ce que** le mélange à sec de poudre initial, la granulation, l'enrobage et le séchage sont réalisés en lit fluidisé.

17. Granule enrobé d'une suspension comprenant au moins un agent d'enrobage et un agent désintégrant membranaire sous forme d'une pellicule, susceptible d'être obtenu par le procédé objet d'une des revendications 1 à 16, **caractérisé en ce que** l'agent désintégrant membranaire et l'agent désintégrant granulaire sont identiques.

18. Granule enrobé selon la revendication 17, **caractérisé en ce qu'**il comprend en poids du granule enrobé :
- de 5 à 70 % d'un polymère d'enrobage,
- de 0,5 à 15 % d'un agent désintégrant granulaire,
- de 1 à 20 % d'un agent désintégrant membranaire,
- de 1 à 20 % d'un agent perméabilisant.

19. Granule enrobé selon la revendication 18, **caractérisé en ce qu'**il comprend en outre de 1 à 20 % en poids d'un agent édulcorant.

20. Granule enrobé selon la revendication 19, **caractérisé en ce qu'**il comprend en poids du granule enrobé :
- de 10 à 40 % d'éthyl cellulose,
- de 3 à 10 % de crospovidone,
- de 2 à 10 % de polyéthylène glycol,
- de 2 à 10 % d'aspartam.

21. Comprimé multiparticulaire à délitement rapide comprenant les granules selon l'une des revendications 17 à 20, **caractérisé en ce que** les granules sont à goût masqué et libération immédiate du principe actif.

22. Comprimé à hydrodispersibilité rapide comprenant les granules selon l'une des revendications 17 à 20, **caractérisé en ce que** les granules sont à goût masqué et libération immédiate du principe actif.

## Claims

1. A process for manufacturing coated granules with masked taste, and immediate release of the active principle, according to which:
• the constituents of a powder comprising at least the active principle and a granular disintegrant are first dry-mixed;
• the powder obtained is then granulated, in the presence of a mixture of excipients comprising at least one binder capable of binding the particles together to give grains and containing no membrane disintegrant;
• the grains formed are then coated by spraying with a suspension comprising at least one coating agent and a membrane disintegrant;
• finally, the coated granules obtained are dried.

2. A process for manufacturing coated granules with masked taste, and immediate release of the active principle, according to which:
• the constituents of a powder comprising at least the active principle and a granular disintegrant are first dry-mixed;
• the powder obtained is then granulated, and the grains formed are then coated, in the presence of the same mixture of excipients comprising at least one binder capable of binding particles together to give grains; at least one coating agent and a membrane disintegrant; the rate of spraying of the mixture of excipients being higher during the granulation step than during the coating step, and the atomization pressure of the mixture of excipients being lower during the granulation step than during the coating step;
• finally, the coated granules obtained are dried.

3. The manufacturing process as claimed in claim 2, **characterized in that** between 10 and 20% of the mixture of excipients is sprayed during the granulation step, the remainder being sprayed during the coating step.

4. The process for manufacturing coated granules as claimed in one of claims 1 to 3, **characterized in that** the granule and membrane disintegrants are high-performance disintegrants chosen from the group comprising sodium carboxymethylcellulose, crospovidone and carboxymethylstarch.

5. The process for manufacturing coated granules as claimed in one of claims 1 to 3, **characterized in that** the binder and the coating agent are chosen from the group comprising cellulose polymers and acrylic polymers.

6. The process for manufacturing coated granules as claimed in claim 5, **characterized in that** the cellulose polymer is chosen from the group comprising ethylcellulose, hydroxypropylcellulose (HPC), carboxymethylcellulose (CMC) and hydroxypropylmethylcellulose (HPMC), alone or as a mixture.

7. The process for manufacturing coated granules as claimed in claim 5, **characterized in that** the acrylic polymer is chosen from the group comprising acrylic polymers and methacrylic polymers, ammonio-methacrylate copolymer, polyacrylate and methacrylic acid copolymer.

8. The process for manufacturing coated granules as claimed in claim 1, **characterized in that** the suspension also comprises a permeabilizer.

9. The process for manufacturing coated granules as claimed in claim 1, **characterized in that** the mixture of excipients used in the granulation step also comprises a permeabilizer.

10. The manufacturing process as claimed in either of claims 2 and 3, **characterized in that** the mixture of excipients also comprises a permeabilizer.

11. The process for manufacturing coated granules as claimed in one of claims 8 to 10, **characterized in that** the permeabilizer is chosen from the group comprising povidone and its derivatives, polyethylene glycol, silica, polyols and low-viscosity cellulose polymers.

12. The process for manufacturing coated granules as claimed in claim 1, **characterized in that** the suspension sprayed during the coating step also comprises a sweetener.

13. The process for manufacturing coated granules as claimed in one of claims 1 to 3, **characterized in that** the dry mix of initial powder also comprises a sweetener.

14. The manufacturing process as claimed in either of claims 2 or 3, **characterized in that** the mixture of excipients also comprises a sweetener.

15. The process for manufacturing coated granules as claimed in either of claims 12 and 14, **characterized in that** the sweetener is chosen from the group comprising aspartam, potassium acesulfam, sodium saccharinate, neohesperidine dihydrochalcone, monoammonium glycyrrhizinate, sugars and derivatives, and also polyols and derivatives, alone or as a mixture.

16. The process for manufacturing coated granules as claimed in claim 1 to 3, **characterized in that** the dry-mixing of initial powder, the granulation, the coating and the drying are performed in a fluidized bed.

17. A coated granule with a suspension comprising at least a binder and a granule disintegrant having a form of a film which may be obtained by the process which is the subject of one of claims 1 to 16 **characterized in that** membrane disintegrant and granule disintegrant are identical.

18. The coated granule as claimed in claim 17, **characterized in that** it comprises, by weight of the coated granule:
- from 5 to 70% of a coating polymer,
- from 0.5 to 15% of a granule disintegrant,
- from 1 to 20% of a membrane disintegrant,
- from 1 to 20% of a permeabilizer.

19. The coated granule as claimed in claim 18, **characterized in that** it also comprises from 1 to 20% by weight of a sweetener.

20. The coated granule as claimed in claim 19, **characterized in that** it comprises, by weight of the coated granule:
- from 10 to 40% ethylcellulose,
- from 3 to 10% crospovidone,
- from 2 to 10% polyethylene glycol,
- from 2 to 10% aspartam.

21. A fast-crumbling multiparticulate tablet comprising the granules which are the subject of one of claims 17 to 20, **characterized in that** granules are with masked taste, and immediate release of the active principle.

22. A fast-dispersible tablet comprising the granules which are the subject of one of claims 17 to 20 **characterized in that** granules are with masked taste, and immediate release of the active principle.

## Patentansprüche

1. Verfahren zur Herstellung eines geschmacksneutral umhüllten Granulats mit sofortiger Freisetzung des Wirkstoffs, gemäß dem:
• zuerst die Bestandteile eines Pulvers trocken gemischt werden, das mindestens den Wirkstoff und ein Granulataufspaltungsmittel umfasst;
• dann das erhaltene Pulver im Beisein eines Arzneimittelträgergemischs granuliert wird, das mindestens ein Bindemittel umfasst, das sich dazu eignet, die Partikel miteinander zu verbinden, um Granulat zu erhalten, und das kein Membranaufspaltungsmittel enthält;
• dann das entstandene Granulat durch Zerstäuben einer Suspension umhüllt wird, die mindestens ein Umhüllungsmittel und ein Membranaufspaltungsmittel umfasst;
• schließlich das erhaltene umhüllte Granulat getrocknet wird.

2. Verfahren zur Herstellung von geschmacksneutral umhüllten Granulats mit sofortiger Freisetzung des Wirkstoffs, gemäß dem:
• zuerst die Bestandteile eines Pulvers trocken gemischt werden, das mindestens den Wirkstoff und ein Granulataufspaltungsmittel umfasst;
• dann das erhaltene Pulver granuliert und anschließend das entstandene Granulat im Beisein eines Arzneimittelträgergemischs umhüllt wird, das mindestens ein Bindemittel umfasst, das sich dazu eignet, die Partikel miteinander zu verbinden, um Granulat zu erhalten; mindestens ein Umhüllungsmittel und ein Membranaufspaltungsmittel; wobei der Zerstäubungsaustrag des Arzneimittelträgergemischs beim Granulierungsschritt höher ist als beim Umhüllungsschritt, und der Zerstäubungsdruck des Arzneimittelträgergemischs beim Granulierungsschritt weniger hoch ist als beim Umhüllungsschritt;
• schließlich das erhaltene umhüllte Granulat getrocknet wird.

3. Herstellungsverfahren nach Anspruch 2, **dadurch gekennzeichnet, dass** zwischen 10 und 20% des Arzneimittelträgergemischs während des Granulierungsschritts zerstäubt werden, wobei der Rest während des Umhüllungsschritts zerstäubt wird.

4. Verfahren zur Herstellung umhüllten Granulats nach einem der Ansprüche 1 bis 3, **dadurch gekennzeichnet, dass** die Granulat- und Membranaufspaltungsmittel hoch wirksame Aufspaltungsmittel sind, die aus der Gruppe ausgewählt sind, die aus Natriumcarboxymethylcellulose, Crospovidon und Carboxymethylamidon besteht.

5. Verfahren zur Herstellung umhüllten Granulats nach einem der Ansprüche 1 bis 3, **dadurch gekennzeichnet, dass** das Bindemittel und das Umhüllungsmittel aus der Gruppe ausgewählt sind, die Cellulosepolymere und Acrylpolymere umfasst.

6. Verfahren zur Herstellung umhüllten Granulats nach Anspruch 5, **dadurch gekennzeichnet, dass** das Cellulosepolymer aus der Gruppe ausgewählt ist, die Ethylcellulose, Hydroxypropylcellulose (HPC), Carboxymethylcellulose (CMC) und Hydroxypropylmethylcellulose (HPMC) allein oder gemischt umfasst.

7. Verfahren zur Herstellung umhüllten Granulats nach Anspruch 5, **dadurch gekennzeichnet, dass** das Acrylpolymer aus der Gruppe ausgewählt ist, die Acrylpolymere und Methacrylpolymere, Ammoniummethacrylat-Copolymer, Polyacrylat, Methacrylsäure-Copolymer umfasst.

8. Verfahren zur Herstellung umhüllten Granulats nach Anspruch 1, **dadurch gekennzeichnet, dass** die Suspension darüber hinaus einen Permeabilitätsförderer umfasst.

9. Verfahren zur Herstellung umhüllten Granulats nach Anspruch 1, **dadurch gekennzeichnet, dass** das Arzneimittelträgergemisch, das im Granulierungsschritt eingesetzt wird, darüber hinaus einen Permeabilitätsförderer umfasst.

10. Verfahren nach einem der Ansprüche 2 oder 3, **dadurch gekennzeichnet, dass** das Arzneimittelträgergemisch darüber hinaus einen Permeabilitätsförderer umfasst.

11. Verfahren zur Herstellung umhüllten Granulats nach einem der Ansprüche 8 bis 10, **dadurch gekennzeichnet, dass** der Permeabilitätsförderer aus der Gruppe ausgewählt ist, die Povidon und dessen Derivate, Polyethylenglycol, Siliziumoxid, Polyole und Cellulosepolymere mit geringer Viskosität umfasst.

12. Verfahren zur Herstellung umhüllten Granulats nach Anspruch 1, **dadurch gekennzeichnet, dass** die beim Umhüllungsschritt zerstäubte Suspension darüber hinaus ein Süßungsmittel umfasst.

13. Verfahren zur Herstellung umhüllten Granulats nach einem der Ansprüche 1 bis 3, **dadurch gekennzeichnet, dass** die Trockenmischung des Ausgangspulvers darüber hinaus ein Süßungsmittel umfasst.

14. Verfahren zur Herstellung umhüllten Granulats nach einem der Ansprüche 2 oder 3, **dadurch gekennzeichnet, dass** das Arzneimittelträgergemisch darüber hinaus ein Süßungsmittel umfasst.

15. Verfahren zur Herstellung umhüllten Granulats nach einem der Ansprüche 12 bis 14, **dadurch gekennzeichnet, dass** das Süßungsmittel aus der Gruppe ausgewählt ist, die Aspartam, Kaliumacesulfam, Natriumsaccharinat, Neohesperidin-Dihydrochalkon, Monoammonium-Glycyrrhizinat, Zucker und Derivate, sowie Polyole und Derivate, allein oder gemischt umfasst.

16. Verfahren zur Herstellung umhüllten Granulats nach einem der Ansprüche 1 bis 3, **dadurch gekennzeichnet, dass** die Trockenmischung des Ausgangspulvers, die Granulierung, die Umhüllung und das Trocknen im Fließbett stattfinden.

17. Granulat, das mit einer Suspension umhüllt ist, die mindestens ein Umhüllungsmittel und ein Membranaufspaltungsmittel in Form einer Haut umfasst, das durch das Verfahren nach einem der Ansprüche 1 bis 16 gewonnen werden kann, **dadurch gekennzeichnet, dass** das Membranaufspaltungsmittel und das Granulataufspaltungsmittel identisch sind.

18. Umhülltes Granulat nach Anspruch 17, **dadurch gekennzeichnet, dass** es in Gewichtsanteilen des umhüllten Granulats umfasst:
- 5 bis 70% eines Umhüllungspolymers,
- 0,5 bis 15% eines Granulataufspaltungsmittels,
- 1 bis 20% eines Membranaufspaltungsmittels,
- 1 bis 20% eines Permeabilitätsförderers.

19. Umhülltes Granulat nach Anspruch 18, **dadurch gekennzeichnet, dass** es darüber hinaus 1 bis 20 Gew.% eines Süßungsmittels umfasst.

20. Umhülltes Granulat nach Anspruch 19, **dadurch gekennzeichnet, dass** es in Gewichtsanteilen des umhüllten Granulats umfasst:
- 10 bis 40% Ethylcellulose
- 3 bis 10% Crospovidon
- 2 bis 10% Polyethylenglycol
- 2 bis 10% Aspartam.

21. Multipartikuläre Tablette mit Schnellspaltung, das Granulat nach einem der Ansprüche 17 bis 20 umfassend, **dadurch gekennzeichnet, dass** das Granulat geschmacksneutral ist und den Wirkstoff sofort freisetzt.

22. Tablette mit schneller Wasserdispersionfähigkeit, das Granulat nach einem der Ansprüche 17 bis 20 umfassend, **dadurch gekennzeichnet, dass** das Granulat geschmacksneutral ist und den Wirkstoff sofort freisetzt.
